# EUROPEAN PATENT SPECIFICATION

(11) **EP 1 427 395 B1**
(45) Date of publication and mention of the grant of the patent: **12.03.2008**
(21) Application number: 02775709.5
(22) Date of filing: 13.09.2002
(51) Int. Cl.: A61K 9/14, A61K 31/337, A61K 31/436

(54) **STABILIZATION OF ACTIVE AGENTS BY FORMULATION INTO NANOPARTICULATE FORM**
STABILISIERUNG AKTIVER WIRKSTOFFE DURCH FORMULIERUNG ALS NANOPARTIKULÄRE ZUSAMMENSETZUNGEN
STABILISATION D'AGENTS ACTIFS PAR FORMULATION SOUS FORME NANOPARTICULAIRE

(30) Priority: 14.09.2001 US 952032
(43) Date of publication of application: 16.06.2004
(60) Divisional application: 07011731.2
(73) Proprietor: Elan Pharma International Limited, Shannon, County Clare (IE)
(72) Inventor: MERISKO-LIVERSIDGE, Elaine, West Chester, PA 19380 (US); WEI, Linden, Exton, PA 19341 (US)
(74) Representative: Khoo, Chong-Yee
(86) International application number: PCT/US2002/025979
(87) International publication number: WO 2003/024424

(56) References cited:
- WO-A-01/26635
- WO-A-99/49846
- US-A- 5 399 363
- US-A- 5 494 683
- US-A- 5 989 591
- US-B1- 6 267 989

## Description

### FIELD

The present invention is directed to methods for chemically stabilizing placlitaxel and drugs containing a macrocyclic lactone ring comprising formulating the active agent into a nanoparticulate composition. The nanoparticulate composition comprises the agent and one or more surface stabilizers adhered to the surface of the active agent.

### BACKGROUND

### A. Summary of Instability and/or Degradation of Active Agents

Active agents are subject to degradation. Such decomposition or degradation may be due to hydrolysis, oxidation, isomerization, epimerization, or photolysis. The rate of degradation or decomposition varies considerably depending on the structural, physical, and chemical nature of the active agent. The rate of decomposition is also often significantly affected by numerous environmental factors, including temperature, light, radiation, enzyme or other catalysts, pH and ionic strength of the solution, solvent type, and buffer species.

Chemical instability due to degradation or decomposition is highly undesirable for several reasons. For example, when a chemical compound is a pharmaceutical agent, degradation decreases its efficiency and shortens its effective shelf life. Moreover, the decrease in the content of the active ingredient in a pharmaceutical preparation renders the calculation of an effective dosage unpredictable and difficult. Furthermore, degraded chemical agent may have highly undesirable or even severely toxic side effects.

Because chemical stability is a critical aspect in the design and manufacture, as well as regulatory review and approval, of pharmaceutical compositions and dosage forms, in recent years extensive and systematic studies have been conducted on the mechanisms and kinetics of decomposition of pharmaceutical agents. For a brief review, see Alfred Martin Physical Pharmacy: Physical Chemical Principles in the Pharmaceutical Sciences, 4th Edition, pp. 305-312 (Lee & Febiger, Philadelphia, 1993).

### B. Prior Methods for Increasing the Stability of a Chemical Compound in Pharmaceutically Acceptable Formulations

Various methods have been devised to achieve improved chemical stability of a compound, including alteration of environmental parameters, such as buffer type, pH, storage temperature, and elimination of catalytic ions or ions necessary for enzyme activity using chelating agents.

Other methods include converting the drug into a more stable prodrug which, under physiological conditions, is processed to become a biologically active form of the compound.

Another method for improving the chemical stability of pharmaceutical agents employs novel dosage form designs. Dosage form designs that improve the chemical stability of a drug include loading drugs into liposomes or polymers, e.g., during emulsion polymerization. However, such techniques have problems and limitations. For example, a lipid soluble drug is often required to prepare a suitable liposome. Further, unacceptably large amounts of the liposome or polymer may be required to prepare unit drug doses. Further still, techniques for preparing such pharmaceutical compositions tend to be complex. Finally, removal of contaminants at the end of the emulsion polymerization manufacturing process, such as potentially toxic unreacted monomer or initiator, can be difficult and expensive.

Another example of a dosage form that can be used to increase the stability of an administered agent is a monolithic device, which is a rate-controlling polymer matrix throughout which a drug is dissolved or dispersed. Yet another example of such a dosage form is a reservoir device, which is a shell-like dosage form having a drug contained within a rate-controlling membrane.

An exemplary reservoir dosage form is described in U.S. Patent No. 4,725,442, which refers to water insoluble drug materials solubilized in an organic liquid and incorporated in microcapsules of phospholipids. One disadvantage of this dosage form is the toxic effects of the solubilizing organic liquids. Other methods of forming reservoir dosage forms of pharmaceutical drug microcapsules include micronizing a slightly-soluble drug by high-speed stirring or impact comminution of a mixture of the drug and a sugar or sugar alcohol together with suitable excipients or diluents. *See e.g.* EP 411,629A. One disadvantage of this method is that the resultant drug particles are larger than those obtained with milling. Yet another method of forming a reservoir dosage form is directed to polymerization of a monomer in the presence of an active drug material and a surfactant to produce small-particle microencapsulation *(*International Journal of Pharmaceutics, 52:101-108 (1989)). This process, however, produces compositions containing contaminants, such as toxic monomers, which are difficult to remove. Complete removal of such monomers can be expensive, particularly when conducted on a manufacturing scale. A reservoir dosage form can also be formed by co-dispersion of a drug or a pharmaceutical agent in water with droplets of a carbohydrate polymer (*see e.g.* U.S. Patent No. 4,713,249 and WO 84/00294). The major disadvantage of this procedure is that in many cases, a solubilizing organic co-solvent is required for the encapsulation procedure. Removal of traces of such harmful co-solvents can result in an expensive manufacturing process.

### C. Background Regarding Nanoparticulate Compositions

Nanoparticulate compositions, first described in U.S. Patent No. 5,145,684 ("the '684 patent"), are particles consisting of a poorly soluble active agent having adsorbed onto the surface thereof a non-crosslinked surface stabilizer. The '684 patent also describes methods of making such nanoparticulate compositions. The '684 patent teaches that nanoparticulate compositions are desirable because with a decrease in particle size, and a consequent increase in surface area, a composition can exhibit superior bioavailability.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer," 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form ofNanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive nanoparticulate compositions having cationic surface stabilizers," and 6,432,381 for "Methods for targeting drag delivery to the upper and/or lower gastrointestinal tract". In addition, U.S. Patent Application No. 20020012675 A1, published on January 31, 2002, for "Controlled Release Nanopartioulate Compositions," describes nanoparticulate compositions.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

In particular, U.S. Patent Nos 5,399,363 and 5,494,683, both for "Surface Modified Anticancer Nanoparticles." refer to nanoparticulate compositions of anticancer agents, including taxol, also known as paclitaxel. However, these patents do not address a means to overcome the chemical instability of an active agent, such as paclitaxel at a basic pH. Specifically, the two patents teach methods of making nanoparticulate paclitaxel compositions which are stable at a pH of 7.4. However, this does not teach or suggest that a nanoparticulate paclitaxel composition is stable at an elevated pH, such as a basic pH of 9.

These references describe methods of increasing the bioavailability of poorly soluble active agents, in which the class of "active agents" encompassed is limited only by the solubility of the active agent. In contrast, the present invention is directed to a class of poorly soluble drugs which are unstable under one or more specified environmental conditions. The two classes of active agents are not the same, as all poorly soluble active agents are not unstable under one or more environmental conditions.

This is significant as the pH varies along the gastrointestinal tract (GIT). If a cancer is localized in a region of the (GIT which has a basic pH, the conventional form ofpaclitaxel will be ineffective, or poorly effective, as the drug degrades at a high pH. This problem can be addressed by nanoparticulate forms of paclitaxel, which are stable at a high pH.

U.S. Patent No. 5,989,591 to Nagi, for "Rapamycin Formulations for Oral Administration," refers to solid dose compositions of rapamycin in which the rapamycin can have a nanoparticulate particle size. *See* Nagi at col. 6, lines 50-55. However, Nagi does not teach that the nanoparticulate rapamycin composition is stable when exposed to an aqueous environment. In fact, Nagi teaches that the nanoparticulate rapamycin composition is formulated into a tablet dosage form, in which rapamycin is present in a sugar coating over a solid core. *See* the Abstract and col. 3, lines 45-52.

This does not teach or suggest that an unstable form of rapamycin, *i*.*e*., rapamycin exposed to an aqueous environment, could be selected and made stable under that same environment by formulating the drug into a nanoparticulate composition. This is significant as tablet dosage forms have a longer onset of activity as compared to liquid or IV dosage forms. In addition, many infant and elderly patients are unable to swallow tablet dosage forms.

U.S. Patent No. 6,267,989 discloses methods for presenting crystal growth and particle aggregation in nanoparticulate compositions, by use of nanoparticulate compositions milled to an optimal effective average particle size from about 150 nm to about 350 nm. International Patent Publication No. WO 99/49846 describes the use of combinations of natural or synthetic phospholipids, a charged surface modifier such as a highly purified charge phospholipid and a block copolymer to physically stabilize particles of compounds to prevent them from particle growth, aggregation or flocculation. Thus, both US 6,267,989 and WO 99/49846 describe methods of physically stabilizing particle containing compositions to prevent particle growth, crystal growth and aggregation of the particles.

There is a need in the art for a method of stabilizing active agents, which is efficient, cost-effective, and does not require the addition of potentially toxic solvents. The present invention satisfies this need.

### SUMMARY

The present invention is based on the discovery that certain active agents which are unstable under one or more environmental conditions, when formulated into nanoparticulate compositions, are stable under those same environmental conditions. Such environmental conditions may include chemical degradation over time, exposure to a non-physiological pH and exposure to solvent molecules.

According to a 1^{st} aspect of the present invention, we provide a method for chemically stabilizing and thereby reducing the rate of degradation of a poorly soluble drug selected from paclitaxel and a drug containing a macrocyclic lactone ring, the method comprising formulating particles of the drug into a stable nanoparticulate drug composition by a method comprising combining the drug particles with at least one non-crosslinked surface stabilizer, wherein after said combining the at least one non-crosslinked surface stabilizer adsorbs to the surface of the drug particles, and said drug particles have an effective average particle size of less than about 2 microns.

The drug may be present in an amount selected from the group consisting of from about 99.5% to about 0.001%, from about 95% to about 0.1%, and from about 90% to about 0.5%, by weight, based on the total combined weight of the at least one drug and at least one surface stabilizer, not including other excipients. The at least one surface stabilizer may be present in an amount selected from the group consisting of from about 0.001 to about 99.5%, from about 0.1% to about 95%, and from about 0.5% to about 90%, by weight, based on the total combined weight of the at least one drug and at least one surface stabilizer, not including other excipients. The nanoparticulate drug composition may have an effective average particle size of less than about 1 micron, less than about 600 nm, less than about 500 nm, less than about 400 run, less than about 300 nm, less than about 200 nm, less than about 100 nm, or less than about 50 nm. The nanoparticulate drug composition may be in an injectable formulation. The drug may be rapamycin. The rapamycin may be stable following exposure to hydrolysis conditions. The drug may be paclitaxel. The paclitaxel may be stable following exposure to basic pH conditions. The drug particles may be in a crystalline phase or in an amorphous phase.

The invention is directed to a process for chemically stabilizing and thereby reducing the rate of degradation of a poorly soluble drug selected from paclitaxel and drug containing a macrocyclic lactone ring, comprising formulating particles of the drug into a stable nanoparticulate composition. The nanoparticulate composition comprises the poorly soluble drug and one or more non-crosslinked surface stabilizers adsorbed on to the surface of the drug. The nanoparticulate compositions have an effective average particle size of less than about two microns.

Preferably, the process is a process for stabilizing rapamycin, which is unstable when exposed to water, comprising forming a nanoparticulate formulation of rapamycin having one or more non-crosslinked surface stabilizers adsorbed on to the surface of the drug. The resultant nanoparticulate rapamycin composition exhibits dramatically superior stability when exposed to water. The pharmaceutical composition preferably comprises a pharmaceutically acceptable carrier, as well as any desired excipients.

The process may also be used for stabilizing paclitaxel, which is unstable when exposed to a basic pH, comprising forming a nanoparticulate composition of paclitaxel having one or more non-crosslinked surface stabilizers adsorbed on to the surface of the drug. The resultant nanoparticulate paclitaxel composition exhibits dramatically superior stability when exposed to a basic pH. The pharmaceutical composition preferably comprises a pharmaceutically acceptable carrier, as well as any desired excipients.

### BRIEF DESCRIPTION OF THE FIGURE

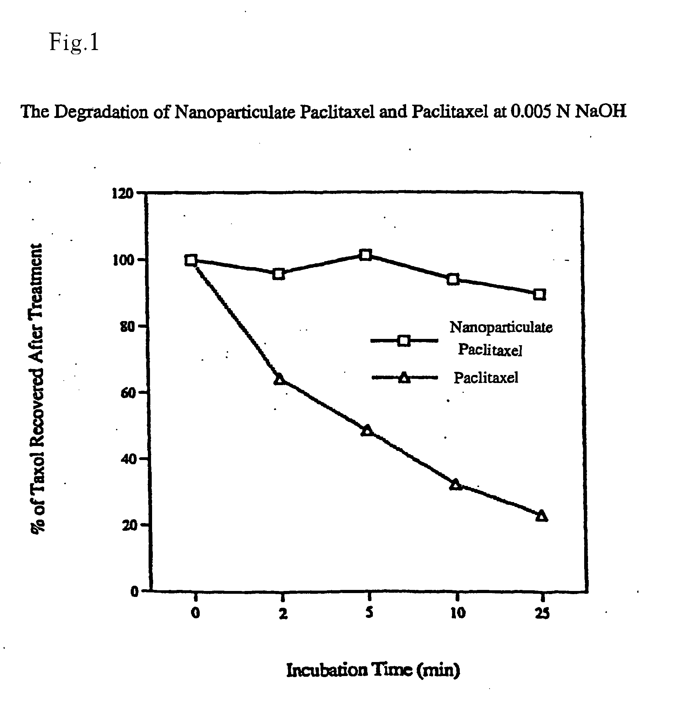

- Figure 1:: Shows the effect of 0.005 N NaOH (a basic pH level) on the rate of degradation of paclitaxel as compared to the rate of degradation of a nanoparticulate formulation of paclitaxel.

### DETAILED DESCRIPTION

The present invention is directed to a method for stabilizing a poorly soluble drug selected from paclitaxel and a drig containing a macrocyclic lactone ring, in order to reduce the rate of degradation of the drug, comprising formulating a drug into a nanoparticulate composition. The nanoparticulate drug is stable under the same one or more environmental conditions under which the drug is unstable prior to formulation into a nanoparticulate composition. Such one or more environmental conditions which can cause the drug to degrade include chemical degradation over time, exposure to non-physiological pH and exposure to solvent molecules.

The method comprises formulating particles of the drug into a stable nanoparticulate composition. The formulation step comprises combining the drug particles and at least one non-crosslinked surface stabilizer, wherein after combining the at least one non-crosslinked surface stabilizer adsorbs to the surface of the drug particles to produce a nanoparticulate composition in which the drug particles have an effective average particle size of less than about 2 microns.

The claimed method is distinct from prior art methods of making nanoparticulate compositions, as prior methods did not teach that a drug selected from paclitaxel and a drug containing a macrocyclic lactone ring can be made stable by formulating the drug into a nanoparticulate composition. Rather, prior methods of making nanoparticulate compositions teach that the nanoparticulate compositions are desirable as they can increase the bioavailability of the component active agent. *See e.g.,* U.S. Patent No. 5,145,684.

The present invention allows the use of these drugs which may have only been useable under limited conditions prior to the present invention. For example, paclitaxel, a drug which is stable only under certain pH conditions, can be formulated for administration at any desired pH level. This is desirable as the pH varies along the human gastrointestinal tract. Similarly, drugs which are unstable when exposed to elevated temperatures can be heat sterilized without degradation when formulated into a nanoparticulate composition. This can be useful in administering drugs to very young or immimocompromised patients. Moreover, rapamycin, a drug which is unstable when exposed to water *(i.e.,* hydrolysis), can be formulated for administration in a liquid form. This can be beneficial as tablet dosage forms have a longer onset of activity as compared to liquid or IV dosage forms. In addition, many infant and elderly patients are unable to swallow tablet dosage forms.

### A. Active Agents and Drags Formulated into Nanoparticulate Compositions Exhibit Increased Stability of the Component Active Agent

Chemical instability due to degradation is usually a result of hydrolysis, oxidation, isomerization, epimerization, or photolysis. Apart from the structural, physical and chemical nature of the active agent, the rate of degradation is often determined by numerous environmental factors, including temperature, light, radiation, enzyme or other catalysts, pH and ionic strength of the solution, solvent type, or buffer species. While not intending to be bound by theory, one possibility is that the molecules of the surface stabilizer shield the drug, thereby protecting potentially labile chemical groups of the active agent from the potentially hostile environment. Another possibility is that for a crystalline drug particle, the crystalline structure in a nanoparticulate sized formulation results in greater active agent stability.

For example, rapamycin is rapidly degraded when exposed to an aqueous environment. The main degradation scheme of rapamycin is the cleavage of the macrocyclic lactone ring by the hydrolysis of an ester bond to form a secoacid (SECO). The secoacid undergoes further dehydration and isomerization to form diketomorpholine analogs.

However, as described in the examples below, when rapamycin is formulated into a nanoparticulate composition, minimal or no rapamycin degradation is observed, even following prolonged exposure to an aqueous medium.

Another example of a drug that is unstable under certain environmental conditions, but which is stable in a nanoparticulate formulation under those same environmental conditions, is paclitaxel. Upon exposure to a basic pH (*i*.*e*., a pH of about 9), paclitaxel rapidly degrades. Ringel et al., J. Pharmac. Exp. Ther., 242:692-698 (1987). However, when paclitaxel is formulated into a nanoparticulate composition, minimal or no paclitaxel degradation is observed, even when the composition is exposed to a basic pH.

### B. Methods of Preparing, Nanoparticulate Compositions

### 1. Drug and Surface Stabilizer Components

The method of stabilizing a drug comprises formulating the drug into a nanoparticulate formulation. The nanoparticulate active agent formulation comprises at least one drug and one or more surface stabilizers adsorbed to the surface of the drug(s).

### a. Drug Particles

The nanoparticles comprise a drug, selected from paclitaxel and a drug containing a macrocyclic lactone ring. The drug has one or more labile groups or exhibiting chemical instability when exposed to certain environmental conditions, such as prolonged storage periods, elevated temperature, non-physiological pH, light, radiation, radiation causing photolysis, enzymes or other catalysts, water (which can cause hydrolysis), solvent molecules, oxidizing agents, free radicals, and freezing-thawing temperature cycles. The drugs may have for example, cosmetic, diagnostic, or bioengineering uses.

The drug may be present either substantially in the form of one optically pure enantiomer or as a mixture, racemic or otherwise, of enantiomers.

A therapeutic drug can be a pharmaceutical, including biologics such as proteins and peptides, and a diagnostic drug is typically a contrast agent such as an x-ray contrast agent, or any other type of diagnostic material. The drug particle exists as a discrete, crystalline phase, amorphous phase, semi-crystalline phase, semi-amorphous phase, or as a combination thereof. The crystalline phase differs from a non-crystalline or amorphous phase which results from precipitation techniques, such as those described in EP Patent No. 275,796.

The drug is preferably present in an essentially pure form, is poorly soluble, and is dispersible in at least one liquid medium. By "poorly soluble" it is mean that the drug has a solubility in a liquid dispersion medium of less than about 30 mg/mL, less than about 10 mg/mL, or less than about 1 mg/mL. The dispersion medium can be, for example, water, safflower oil, ethanol, t-butanol, glycerin, polyethylene glycol (PEG), hexane, or glycol.

The drug is selected from paclitaxel and a drug containing a macrocyclic lactone ring.

### b. Surface Stabilizers

Exemplary useful surface stabilizers include, but are not limited to, known organic and inorganic pharmaceutical excipients. Such excipients include various polymers, low molecular weight oligomers, natural products, and surfactants. Preferred surface stabilizers include nonionic, cationic, and ionic surfactants. Combinations of more than one surface stabilizer can be used in the invention. The surface stabilizers do not chemically interact with the active agent particles, and individually adsorbed molecules of the surface stabilizer are essentially free of intermolecular crosslinkages,

Representative examples of surface stabilizers include hydroxypropyl methylcellulose, hydroxypropylcellulose, polyvinylpyrrolidone, random copolymers of vinyl pyrrolidone and vinyl acetate, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.*, macrogol ethers such as cetomacrogoll 1000), polyoxyethylene castor oil derivatives, polyoxyethylene sorbitan fatty acid esters (*e.g.*, the commercially available Tweens^{®} such as *e.g.,* Tween 20^{®} and Tween 80^{®} (ICI Speciality Chemicals)); polyethylene glycols (*e.g.,* Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g.*, Pluronics F68^{®} and F108^{®}, which are block copolymers of ethylene oxide and propylene oxide); poloxamines (*e.g.*, Tetronic 908^{®}, also known as Poloxamine 908^{®},which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂C(O)N(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄)dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecylirimethylammonium bromide, methyl trioctylammonium chloride (ALIQUAT 336^{™}), POLYQUAT 10^{™}, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quaternized polyoxyethylalkylamines, MIRAPOL^{™} and ALKAQUAT^{™} (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Particularly preferred nonpolymeric primary stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CR₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetallconium chloride, cetrimonium bromide, cetrimonium chloride, cethylamine hydrofluoride, chlorallylmethenamine chloride (Quaternium-15), distearyldimonium chloride (Quatemium-5), dodecyl dimethyl ethylbenzyl ammonium chloride (Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, lofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the *Handbook of Pharmaceutical Excipients,* published jointly by the American Pharmaceutical Association and The Pharmaceutical Society of Great Britain (The Pharmaceutical Press, 2000). The surface stabilizers are commercially available and/or can be prepared by techniques known in the art.

### c. Nanoparticulate Drug/Surface Stabilizer Particle Size

The compositions may contain nanoparticulate drug particles which have an effective average particle size of less than about 2 microns, less than about 1 micron, less than about 600 nm, less than about 500 nm, less than about 400 nm, less than about 300 nm, less than about 200 nm, less than about 100 nm, or less than about 50 nm, as measured by light-scattering methods, microscopy, or other appropriate methods.

By "an effective average particle size of "less than about 2 microns," it is meant that at least 50% of the drug particles have a weight average particle size of less than about 2 microns when measured by light scattering techniques, microscopy, or other appropriate methods. Preferably, at least 70% of the drug particles have an average particle size of less than about 2 microns, more preferably at least 90% of the drug particles have an average particle size of less than about 2 microns, and even more preferably at least about 95% of the particles have a weight average particle size of less than about 2 microns.

### d. Concentration of Nanoparticulate Drug and Surface Stabilizer

The relative amount of drug and one or more surface stabilizers can vary widely. The optimal amount of the one or more surface stabilizers can depend, for example, upon the particular drug selected, the hydrophilic lipophilic balance (HLB), melting point, and water solubility of the surface stabilizer, and the surface tension of water solutions of the surface stabilizer, *etc*.

The concentration of the at least one drug can vary from about 99.5% to about 0.001%, from about 95% to about 0.1%, or from about 90% to about 0.5%, by weight based on the total combined weight of the at least one drug and at least one surface stabilizer, not including other excipients.

The concentration of the at least one surface stabilizer can vary from about 0.001 to about 99.5%, from about 0.1 % to about 95%, and from about 0.5% to about 90%, by weight, based on the total combined weight of the at least one drug and at least one surface stabilizer, not including other excipients.

### 2. Methods of Making Nanoparticulate Formulations

Nanoparticulate drug agent compositions can be made using methods known in the art such as, for example, milling, homogenization, and precipitation techniques. Exemplary methods of making nanoparticulate compositions are described in U.S. Patent No. 5,145,684. Methods of making nanoparticulate compositions are also described in U.S. Patent Nos. 5,518,187; 5,718,388; 5,862,999; 5,665,331; 5,662,883; 5,560,932; 5,543,133; 5,534,270; 5,510,118; and 5,470,583.

### 1. Milling to Obtain Nanoparticulate Active Agent Dispersions

Milling an active agent to obtain a nanoparticulate composition comprises dispersing active agent particles in a liquid dispersion medium in which the active agent is poorly soluble, followed by applying mechanical means in the presence of grinding media to reduce the particle size of the active agent particles to the desired effective average particle size. The dispersion medium can be, for example, water, safflower oil, ethanol, t-butanol, glycerin, polyethylene glycol (PEG), hexane, or glycol.

The active agent particles can be reduced in size in the presence of at least one surface stabilizer. Alternatively, the active agent particles can be contacted with one or more surface stabilizers after attrition. Other compounds, such as a diluent, can be added to the active agent/surface stabilizer composition during the size reduction process. Dispersions can be manufactured continuously or in a batch mode. The resultant nanoparticulate active agent dispersion can be utilized in solid or liquid dosage formulations, such as controlled release formulations, solid dose fast melt formulations, aerosol formulations, lyophilized formulations, tablets, capsules, solid lozenge, powders, etc.

### 2. Precipitation to Obtain Nanoparticulate Active Agent Compositions

Another method of forming the desired nanoparticulate active agent composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving an active agent in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means. The resultant nanoparticulate active agent dispersion can be utilized in solid or liquid dosage formulations, such as controlled release formulations, solid dose fast melt formulations, aerosol formulations, lyophilized formulations, tablets, solid lozenge, powders, capsules, etc.

### 3. Homogenization to Obtain Active Agent Nanoparticulate Compositions

Exemplary homogenization methods of preparing active agent nanoparticulate compositions are described in U.S. Patent No. 5,510,118, for "Process of Preparing Therapeutic Compositions Containing Nanoparticles."

### C. Methods of Using Nanoparticulate Active Agent Formulations

The nanoparticulate compositions made by the methods of the present invention can be administered to humans and animals either orally, rectally, parenterally (intravenous, intramuscular, or subcutaneous), intracisternally, intravaginally, intraperitoneally, locally (powders, ointments or drops), or as a buccal or nasal spray.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate.

Proper fluidity can be maintained, for example, by the use of a coating such as lecithin, by the maintenance of the required particle size in the case of dispersions, and by the use of surfactants. The nanoparticulate compositions may also contain adjuvants, such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include capsules, tablets, pills, powders, and granules. In such solid dosage forms, the active compound is admixed with at least one of the following: (a) one or more inert excipients (or carrier), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active compounds, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

Actual dosage levels of drug m the nanoparticulate compositions of the invention may be varied to obtain an amount of active agent that is effective to obtain a desired therapeutic response for a particular composition and method of administration. The selected dosage level therefore depends upon the desired therapeutic effect on the route of administration, on the desired duration of treatment, and other factors.

The total daily dose of the compounds administered to a host in single or divided dose may be in amounts of, for example, from about 1 nanomole to about 5 micromoles per kilogram of body weight. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other active agents, and the severity of the particular disease being treated. The following examples are given to illustrate the present invention.

### Example 1

The purpose of this example was to determine the effect on the stability of paclitaxel at a basic pH when the drug is formulated into a nanoparticulate composition.

Paclitaxel is a naturally occurring diterpenoid which has demonstrated great potential as an anti-cancer drug. Paclitaxel can be isolated from the bark of the western yew, *Taxus brevifolia,* and is also found in several other yew species such as *T. baccata* and *T. cuspidata.* Upon exposure to a basic pH (*i.e.*, a pH of about 9), the drug rapidly degrades. Ringel et al., J. Pharmac. Exp. Ther., 242:692-698 (1987).

Two formulations of paclitaxel were prepared: a solubilized formulation of paclitaxel and a nanoparticulate formulation of paclitaxel. The degradation of paclitaxel for both formulations was then compared. For Formulation I, paclitaxel (Biolyse; Quebec, Canada) was solubilized in 1% methanol and 99% H₂O to make a 2% paclitaxel solution. Formulation II was prepared by milling the 2% paclitaxel solution with 1 % Plurionic F108^{™} (BASF) in a 0.5 oz amber bottle containing 7.5 ml 0.5mm Yttria-doped Zirconia media on a U.S. Stoneware Roller Mill for 72 hours. The resultant milled paclitaxel composition had an effective average particle size of about 220 nm, as measured by a Coulter Counter (Coulter Electronics Inc.).

Both solubilized paclitaxel (Formulation I) and nanoparticulate paclitaxel (Formulation In were incubated with 0.005 N NaOH solution (a basic solution). At the end of the incubation period, base degradation of paclitaxel was stopped by adding to the incubation solution 1/100 its volume of 1N HCl. The recovery of paclitaxel was then measured at various time periods by HPLC.

As shown in Figure 1, solubilized paclitaxel rapidly degraded when exposed to basic pH conditions, as only about 20% of the paclitaxel was recoverable after a 20 minute incubation period. In contrast, nanoparticulate paclitaxel was essentially stable under basic conditions, as more than 90% of the drug was recoverable after the same incubation period.

### Example 2

The purpose of this example was to determine the stability of rapamycin when the drug is formulated into a nanoparticulate composition.

Rapamycin is useful as an immunosuppressant and as an antifungal antibiotic, and its use is described in, for example, U.S. Patent Nos. 3,929,992, 3,993,749, and 4,316,885, and in Belgian Pat. No. 877,700. The compound, which is only slightly soluble in water, *i*.*e*., 20 micrograms per mL, rapidly hydrolyzes when exposed to water. Because rapamycin is highly unstable when exposed to an aqueous medium, special injectable formulations have been developed for administration to patients, such as those described in European Patent No. EP 041,795. Such formulations are often undesirable, as frequently the non-aqueous solubilizing agent exhibits toxic side effects.

Two different formulations of rapamycin were prepared and then exposed to different environmental conditions. The degradation ofrapamycin for each of the formulations was then compared. The two formulations were prepared as follows:
(1) Formulation I, a mixture of 5% rapamycin and 2.5% Plurionic F68^{™} (BASF) in an aqueous medium; and
(2) Formulation II, a mixture of 5% rapamycin and 1.25% Plurionic F108^{™} (BASF) in an aqueous medium.

Each of the two formulations was milled for 72 hours in a 0.5 ounce bottle containing 0.4 mm Yttria beads (Performance Ceramics Media) on a U.S. Stoneware Mill. Particle sizes of the resultant nanoparticulate compositions were measured by a Coulter Counter (Model No. N4MD). Following milling, Formulations I and II had effective average particle sizes of 162 nm and 171 nm, respectively.

The samples were then diluted to about 2% rapamycin with Water For Injection (WFI), bottled, and then either stored at room temperature or frozen upon completion of milling and then thawed and stored at room temperature. After ten days of storage at room temperature, Formulations I and II had effective average particle sizes of 194 nm and 199 nm, respectively.

The strength of the rapamycin in the formulations was measured by HPLC, the results of which are shown below in Table I.

**TABLE I**

| **Stability of Nanoparticulate Rapamycin under Different Storage Conditions** | | | | | |
|---|---|---|---|---|---|
| Sample | Description | Storage Conditions | Storage Time | Ending Strength/ Starting Strength | SECO %* |
| 1 | Formulation I | RT | 2 days | 97% | <detection limit |
| 2 | Formulation II | RT | 2 days | 99% | <detection limit |
| 3 | Formulation III | RT | 2 days | 96% | <detection limit |
| 7 | Formulation I | Frozen/thawed | 2 days | 95% | <detection limit |
| 8 | Formulation II | Frozen/thawed | 2 days | 98% | <detection limit |
| 9 | Formulation III | Frozen/thawed | 2 days | 97% | <detection limit |
| 1 | Formulation I | RT | 3 wks | 95% | <detection limit |
| 2 | Formulation II | RT | 3 wks | 98% | <detection limit |
| 3 | Formulation III | RT | 3 wks | 98% | <detection limit |

| | | | | | |
|---|---|---|---|---|---|
| *SECO, or secoacid, is the primary degradation product of rapamycin. The detection limit is 0.2%. | | | | | |

The results show that the nanoparticulate rapamycin formulation exhibited minimal degradation of rapamycin following prolonged storage periods or exposure to the environmental conditions of freezing and thawing.

### Example 3

The purpose of this example was to determine the effect of rapamycin concentration on the chemical stability of rapamycin in a nanoparticulate formulation following autoclaving.

Three rapamycin formulations were prepared by milling the following three slurries in a 250 ml Pyrex^{™} bottle containing 125 ml 0.4 mm Yttria-doped Zirconia media for 72 hours on a U.S. Stoneware roller mill:
(a) 5% rapamycin/1.25% Plurionic F68^{™}
(b) 5% rapamycin/2.5% Plurionic F68^{™}
(c) 5% rapamycin/5% Plurionic F68^{™}

Each of the three dispersions was then diluted with water to prepare formulations having rapamycin concentrations of 4.4%, 2.2%, 1.1% and 0.5% as follows:
(1) Formulation 1: a mixture of 4.4% rapamycin and, prior to dilution, 1.25% Plurionic F68^{™} in an aqueous medium;
(2) Formulation 2: a mixture of 4.4% rapamycin and, prior to dilution, 2.5% Plurionic F68^{™} in an aqueous medium;
(3) Formulation 3: a mixture of 4.4% rapamycin and, prior to dilution, 5% Plurionic F68^{™} in an aqueous medium;
(4) Formulation 4: a mixture of 2.2% rapamycin and, prior to dilution, 1.25% Plurionic F68^{™} in an aqueous medium;
(5) Formulation 5: a mixture of 2.2% rapamycin and, prior to dilution, 2.5% Plurionic F68^{™} in an aqueous medium;
(6) Formulation 6: a mixture of 2.2% rapamycin and, prior to dilution, 5% Plurionic F68^{™} in an aqueous medium;
(7) Formulation 7: a mixture of 1.1% rapamycin and, prior to dilution, 1.25% Plurionic F68^{™} in an aqueous medium;
(8) Formulation 8: a mixture of 1.1 % rapamycin and, prior to dilution, 2.5% Plurionic F68^{™} in an aqueous medium;
(9) Formulation 9: a mixture of 1.1 % rapamycin and, prior to dilution, 5% Plurionic F68^{™} in an aqueous medium;
(10) Formulation 10: a mixture of 0.55% rapamycin and, prior to dilution, 1.25% Plurionic F68^{™} in an aqueous medium;
(11) Formulation 11: a mixture of 0.55% rapamycin and, prior to dilution, 2.5% Plurionic F68^{™} in an aqueous medium; and
(12) Formulation 12: a mixture of 0.55% rapamycin and, prior to dilution, 5% Plurionic F68^{™} in an aqueous medium;

All twelve of the nanoparticulate formulations were autoclaved for 25 minutes at 121°C. The formulations were then stored at 4°C for 61 days, followed by testing for rapamycin degradation. No degradation, as measured by the percent of the SECO degradation product, was detected for any of the formulations.

### Example 4

The purpose of this example was to determine the chemical stability of a nanoparticulate rapamycin formulation following a prolonged storage period at room temperature.

A mixture of 20% rapamycin and 10% Plurionic F68™ in an aqueous medium was milled with 0.4 mm YTZ media (Performance Ceramic Co.) on a U.S. Stoneware mill for 72 hours at room temperature. The final nanoparticulate composition had a mean particle size of between 180 to 230 nm, as measured by Coulter sizing.

After two weeks of storage at room temperature, no SECO degradation product was detected in any of the nanoparticulate preparations, indicating that there was minimal or no degradation of rapamycin in the stored nanoparticulate formulation samples.

### Example 5

The purpose of this example was to determine the effect of long term storage on the chemical stability of rapamycin in a nanoparticulate composition.

Three different nanoparticulate rapamycin formulations were prepared as follows: Formulation 1, having a rapamycin concentration of 182.8 mg/mL; Formulation 2, having a rapamycin concentration of 191.4 mg/mL; and Formulation 3, having a rapamycin concentration of 192.7 mg/mL.

The formulations were prepared by milling the following three slurries in a 0.5 oz amber bottle containing 7.5 ml 0.8 mm Yttria-doped Zirconia media for 72 hours on a U.S. Stoneware roller mill:
(1) 20% rapamycin/10% Plurionic F68
(2) 20% rapamycin/5% Plurionic F68
(3) 20% rapamycin/2.5% Plurionic F68

Following storage for two and half months, no SECO degradation product was detected in any of the samples. These results show that various dosage strengths of rapamycin can be used in nanoparticulate formulations without any impact on the increased chemical stability of the drug.

## Claims

1. A method for chemically stabilizing and thereby reducing the rate of degradation of a poorly soluble drug selected from paclitaxel and a drug containing a macrocyclic lactone ring, the method comprising formulating particles of the drug into a stable nanoparticulate drug composition by a method comprising combining the drug particles with at least one non-crosslinked surface stabilizer, wherein after said combining the at least one non-crosslinked surface stabilizer adsorbs to the surface of the drug particles, and said drug particles have an effective average particle size of less than about 2 microns.

2. A method according to Claim 1, wherein the drug is present in an amount selected from the group consisting of from about 99.5% to about 0.001%, from about 95% to about 0.1%, and from about 90% to about 0.5%, by weight, based on the total combined weight of the at least one drug and at least one surface stabilizer, not including other excipients.

3. A method according to Claims 1 or 2, wherein the at least one surface stabilizer is present in an amount selected from the group consisting of from about 0.001 to about 99.5%, from about 0.1% to about 95%, and from about 0.5% to about 90%, by weight, based on the total combined weight of the at least one drug and at least one surface stabilizer, not including other excipients.

4. A method according to any one of Claims 1 to 3, wherein the nanoparticulate drug composition has an effective average particle size of less than about 1 micron, less than about 600 nm, less than about 500 nm, less than about 400 nm, less than about 300 nm, less than about 200 nm, less than about 100 nm, or less about 50 nm.

5. A method according to any one of Claims 1 to 4, wherein the nanoparticulate drug composition is an injectable formulation.

6. A method according to any one of Claims 1 to 5, wherein the drug is rapamycin.

7. A method according to Claim 6, wherein said rapamycin is stable following exposure to hydrolysis conditions.

8. A method according to any one of Claims 1 to 5, wherein the drug is paclitaxel.

9. A method according to Claim 8, wherein said paclitaxel is stable following exposure to basic pH conditions.

10. A method according to any one of Claims 1 to 9, wherein the drug particles are in a crystalline phase or in an amorphous phase.

## Patentansprüche

1. Verfahren zur chemischen Stabilisierung und gleichzeitigen Reduzierung der Geschwindigkeit des Abbaus eines schlecht löslichen Arzneimittels, ausgewählt unter Paclitaxel und einem Arzneimittel, das einen makrozyklischen Lactonring enthält, wobei das Verfahren umfaßt, daß man Teilchen des Arzneimittels in eine stabile nanopartikuläre Arzneimittelzusammensetzung nach einem Verfahren formuliert, bei dem man die Arzneimittelteilchen mit wenigstens einem nicht vernetzten Oberflächenstabilisator vereinigt, wobei der wenigstens eine nicht vernetzte Oberflächenstabilisator nach dieser Vereinigung an die Oberfläche der Arzneimittelteilchen adsorbiert und die Arzneimittelteilchen eine effektive mittlere Teilchengröße von weniger als etwa 2 µm haben.

2. Verfahren nach Anspruch 1, wobei das Arzneimittel in einer Menge vorliegt, die aus der Gruppe ausgewählt ist, bestehend aus von etwa 99,5 Gew. % bis etwa 0,001 Gew. %, von etwa 95 Gew. % bis etwa 0,1 Gew. % und von etwa 90 Gew. % bis etwa 0,5 Gew. %, bezogen auf das gesamte vereinigte Gewicht des wenigstens einen Arzneimittels und des wenigstens einen Oberflächenstabilisators, wobei andere Hilfsstoffe nicht umfaßt sind.

3. Verfahren nach Anspruch 1 oder 2, wobei der wenigstens eine Oberflächenstabilisator In einer Menge vorliegt, die aus der Gruppe ausgewählt ist, bestehend aus von etwa 0,001 bis etwa 99,5 Gew. %, von etwa 0,1 Gew. % bis etwa 95 Gew.% und von etwa 0,5 Gew. % bis etwa 90 Gew. %, bezogen auf das gesamte vereinigte Gewicht des wenigstens einen Arzneimittels und des wenigstens einen Oberflächenstabilisators, wobei andere Hilfsstoffe nicht umfaßt sind.

4. Verfahren nach einem der Ansprüche 1 bis 3, wobei die nanopartikuläre Arzneimittelzusammensetzung eine effektive mittlere Teilchengröße von weniger als etwa 1 µm, weniger als etwa 600 nm, weniger als etwa 500 nm, weniger als etwa 400 nm, weniger als etwa 300 nm, weniger als etwa 200 nm, weniger als 100 nm oder weniger als etwa 50 nm aufweist.

5. Verfahren nach einem der Ansprüche 1 bis 4, wobei die nanopartikuläre Arzneimittelzusammensetzung eine injizierbare Formulierung ist.

6. Verfahren nach einem der Ansprüche 1 bis 5, wobei das Arzneimittel Rapamyzin ist.

7. Verfahren nach Anspruch 6, wobei das Rapamyzin nach dem Aussetzen an Hyprolysebedingungen stabil ist

8. Verfahren nach einem der Ansprüche 1 bis 5, wobei das Arzneimittel Paclitaxel ist.

9. Verfahren nach Anspruch 8, wobei das Paclitaxel nach Aussetzen an Bedingungen von basischem pH-Wert stabil ist.

10. Verfahren nach einem der Ansprüche 1 bis 9, wobei die Arzneimittelteilchen in einer kristallinen Phase oder in einer amorphen Phase vorliegen.

## Revendications

1. Procédé pour stabiliser chimiquement et ainsi réduire la vitesse de dégradation d'un médicament peu soluble choisi parmi le paclitaxel et un médicament contenant un cycle lactonique macrocyclique, le procédé comprenant l'étape consistant à formuler des particules du médicament en une composition de médicament nanoparticulaire stable par un procédé qui comprend la mise en association des particules de médicament avec au moins un stabilisant de surface non réticulé, dans lequel, après ladite mise en association, ledit au moins un stabilisant de surface non réticulé est adsorbé. à la surface des particules de médicament, et lesdites particules de médicament ont une taille moyenne effective de particules inférieure à environ 2 micromètres.

2. Procédé selon la revendication 1, dans lequel le médicament est présent en une quantité choisie dans le groupe d'environ 99,5 % à environ 0,001 %, d'environ 95 % à environ 0,1 % et d'environ 90 % à environ 0,5 % en poids, par rapport au poids combiné total dudit au moins un médicament et dudit au moins un stabilisant de surface, à l'exclusion d'autres excipients.

3. Procédé selon la revendication 1 ou 2, dans lequel ledit au moins un stabilisant de surface est présent en une quantité choisie dans le groupe d'environ 0,001 à environ 99,5 %, d'environ 0,1 % à environ 95 % et d'environ 0,5 % à environ 90 % en poids, par rapport au poids combiné total dudit au moins un médicament et dudit au moins un stabilisant de surface, à l'exclusion d'autres excipients.

4. Procédé selon l'une quelconque des revendications 1 à 3, dans lequel la composition de médicament nanoparticulaire a une taille moyenne effective de particules inférieure à environ 1 micromètre, inférieure à environ 600 nm, inférieure à environ 500 nm, inférieure à environ 400 nm, inférieure à environ 300 nm, inférieure à environ 200 nm, inférieure à environ 100 nm ou inférieure à environ 50 nm.

5. Procédé selon l'une quelconque des revendications 1 à 4, dans lequel la composition de médicament nanoparticulaire est une formulation injectable.

6. Procédé selon l'une quelconque des revendications 1 à 5, dans lequel le médicament est la rapamycine.

7. Procédé selon la revendication 6, dans lequel ladite rapamycine est stable après exposition à des conditions d'hydrolyse.

8. Procédé selon l'une quelconque des revendications 1 à 5, dans lequel le médicament est le paclitaxel.

9. Procédé selon la revendication 8, dans lequel ledit paclitaxel est stable après exposition à des conditions de pH basique.

10. Procédé selon l'une quelconque des revendications 1 à 9, dans lequel les particules de médicament sont dans une phase cristalline ou dans une phase amorphe.
